# EUROPÄISCHE PATENTANMELDUNG

(11) **EP 1 264 825 A1**
(43) Veröffentlichungstag der Anmeldung: **11.12.2002**
(21) Anmeldenummer: 02016939.7
(22) Anmeldetag: 15.11.1996
(51) Int. Cl.: C07D 213/20, C09B 69/06

(54) **Beta-Hydroxyalkylpicoliniumsalze**

(30) Priorität: 28.11.1995 DE 19544268
(62) Teilanmeldung aus: 96118312.6
(71) Anmelder: Bayer Aktiengesellschaft, 51368 Leverkusen (DE)
(72) Erfinder: Meisel, Karlheinrich, Dr., 51519 Odenthal (DE); Walz, Klaus, Dr., 51381 Leverkusen (DE); Renner, Gerd-Friedrich, Dr., 51515 Kürten (DE); Schulze, Hans, Dr., 51061 Köln (DE); Gerdes, Carsten, Dr., 51373 Leverkusen (DE); Gassen, Karl-Rudolf, Dr., 40885 Ratingen (DE); Ditzer, Reiner, Dr., 51519 Odenthal (DE); Klein, Lothar, Dr., 51375 Leverkusen (DE)

(57) **Zusammenfassung**

ß-Hydroxyalkylpicoliniumsalze der Formel (I) 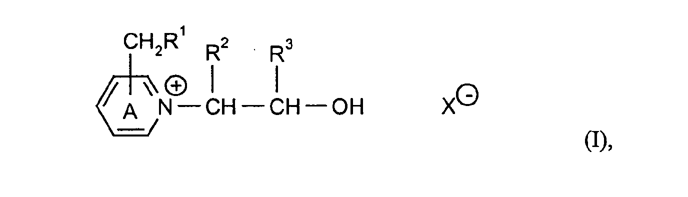 worin A, R¹ bis R³ und X die in der Beschreibung genannten Bedeutungen haben, die durch Umsetzung von Picolinen der Formel (II) 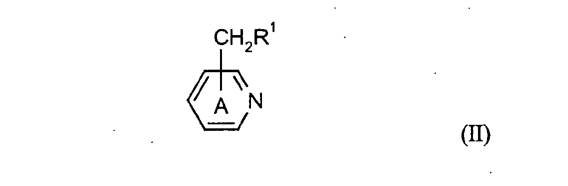 in Wasser oder einem organischen Lösungsmittel als Reaktionsmedium mit Alkylenoxiden der Formel (III) in Gegenwart einer 1- bis 3-basischen Säure erhalten und als Zwischenprodukte zur Herstellung kationischer Farbstoffe verwendet werden sowie solche kationische Farbstoffe enthaltenden Farbstoffpräparationen.

## Beschreibung

Die Erfindung betrifft β-Hydroxyalkylpicoliniumsalze, ein Verfahren zu ihrer Herstellung, ihre Verwendung als Zwischenprodukte zur Herstellung kationischer Farbstoffe sowie Farbstoffpräparationen daraus hergestellter kationischer Farbstoffe mit geringem Chloridgehalt.

Hydroxyethylpicoliniumsalze sind insbesondere in Form ihrer Chloride, die beispielsweise aus EP-A 176 472 bekannt sind, wichtige Zwischenprodukte bei der Herstellung von kationischen Farbstoffen. Die Chloride werden im allgemeinen durch Umsetzung von Chlorethanol mit Picolinen erhalten. Bei diesem Verfahren wird jedoch das problembehaftete, weil toxische und korrosive, Chlorethanol verwendet.

Aufgabe der vorliegenden Erfindung war es, ein Verfahren zur Herstellung von Hydroxyethylpicoliniumsalzen bereitzustellen, das die genannten Nachteile nicht aufweist.

Es wurde nun ein Verfahren zur Herstellung von β-Hydroxyalkylpicoliniumsalzen der Formel (I) 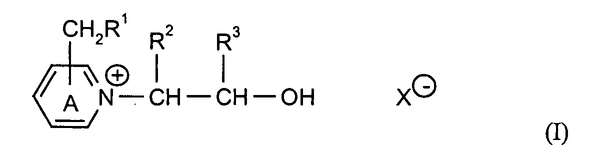 gefunden, wobei der Ring A gegebenenfalls weitere Substituenten trägt und/oder mit carbo- oder heterocyclischen Ringen, vorzugsweise aromatischen, kondensiert sein kann,
- R¹: für H, Cyano, C₁-C₄-Alkoxycarbonyl oder gegebenenfalls substituiertes C₁-C₄-Alkyl, insbesondere C₁-C₂-Alkyl, steht,
- R² und R³: unabhängig voneinander H, gegebenenfalls substituiertes C₁-C₄-Alkyl, insbesondere C₁-C₂-Alkyl, oder gegebenenfalls substituiertes Phenyl bedeuten und
- X⁻: für den Rest eines Äquivalents einer 1- bis 3-basischen Säure steht,
das dadurch gekennzeichnet ist, dass man Picoline der Formel (II) 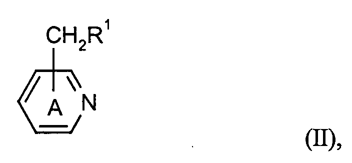 worin R¹ die oben genannte Bedeutung besitzt und der Ring A in oben beschriebener Weise weiter substituiert sein kann, mit Alkylenoxiden der Formel (III) 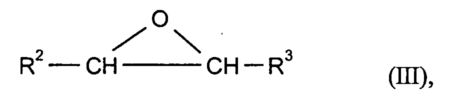 worin R² und R³ die oben genannte Bedeutung besitzen, in Gegenwart einer 1- bis 3-basischen Säure umsetzt.

Als geeignete Substituenten für die C₁-C₄-Alkylreste in der Bedeutung von R² und R³ sind beispielsweise CN, Aryloxy wie Phenoxy oder C₁-C₄-Alkoxycarbonyle zu nennen.

Als geeignete Substituenten für Phenylreste sind beispielsweise C₁-C₄-Alkyl, Halogen, insbesondere Cl, Br und F, CN, NO₂ oder C₁-C₂-Alkylsulfonyl zu nennen.

In einer besonders bevorzugten Ausführungsform des erfindungsgemäßen Verfahrens werden als Alkylenoxide der Formel (III) Ethylenoxid, Propylenoxid, 1,2- oder 2,3-Butylenoxid, Styroloxid oder 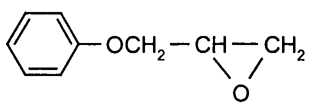 eingesetzt. Ganz besonders bevorzugt ist Ethylenoxid und Propylenoxid, insbesondere Ethylenoxid.

Als bevorzugte Picoline der Formel (II) sind zu nennen: 4-Methylpyridin, 2-Methylpyridin, 4-Methylchinolin, 2-Methylchinolin oder 4-Methyltetrahydrochinolin. Besonders bevorzugt ist 4-Methylpyridin.

Als geeignete weitere Substituenten des Ringes A sind beispielsweise zu nennen: Nitro, Halogen, Cyano, C₁-C₄-Alkyl, C₁-C₄-Alkoxy, Acetylamino oder Dimethylamino. Für den Fall, daß der Ring A mit anderen Ringen kondensiert ist, sind dies vorzugsweise carbocyclische, insbesondere aromatische Benzo- oder Naphthalin-Ringe. Diese sind besonders bevorzugt in 2,3-Stellung des A-Ringes ankondensiert.

Als Reaktionsmedium dient vorzugsweise Wasser oder ein Gemisch aus Wasser und einem polaren organischen Lösungsmittel, wobei das polare organische Lösungsmittel vorzugsweise mit Wasser mischbar, insbesondere in Wasser löslich ist. Als besonders bevorzugte organische Lösungsmittel sind Glykole wie Ethylenglykol, Diethylenglykol, 1,2- und 1,3-Propandiol, Glycerin und Pentaerythrit, Glykolether wie n-Butyl-diethylenglykol, Methoxypropanol, DMF zu nennen. Ganz besonders bevorzugt ist Ethylenglykol oder Propylenglykol (1,2).

Das Mischungsverhältnis von Wasser zu organisch polarem Lösungsmittel kann über große Bereiche variiert werden. Als vorteilhaft hat sich ein Gewichtsverhältnis von 1:3 bis 1:10 erwiesen.

Als geeignete 1- bis 3-basische Säure ist sowohl eine anorganische wie beispielsweise HCl, H₂SO₄, H₃PO₄, HNO₃, HBr, HJ, H₂CO₃ oder eine organische Carbon-, Sulfonoder Sulfinsäure wie beispielsweise C₁-C₆-Mono- oder -Dicarbonsäure wie Ameisensäure, Essigsäure oder Oxalsäure, Hydroxy-C₁-C₄-alkylsulfonsäure oder deren Mischungen zu nennen.

Die Säure kann pur, in konzentrierter oder verdünnter wässriger Lösung zugegeben werden. Säuregemische sind ebenfalls möglich. Besonders bevorzugt ist H₂SO₄, insbesondere konzentriert (48 bis 98 Gew.-%).

Das Anion X⁻ des Hydroxyethylpicoliniumsalzes leitet sich von der 1- bis 3-basischen Säure ab. So ist beispielsweise auch β-Hydroxyethylsulfat als Anion ein im Rahmen dieser Anmeldung verstandener Abkömmling der 2-basischen Säure H₂SO₄, das gebildet wird, wenn als Alkylenoxid Ethylenoxid verwendet wird.

Das erfindungsgemäße Verfahren wird in der Regel bei einer Temperatur von 0 bis 160°C, insbesondere bei 70 bis 100°C durchgeführt. Das erfindungsgemäße Verfahren kann bei Normaldruck, vermindertem oder erhöhtem Druck durchgeführt werden. Vorteilhaft ist es, die Umsetzung bei Normaldruck oder bei erhöhtem Druck, insbesondere bei 3 bis 6 bar durchzuführen. Eine Druckerhöhung ist insbesondere dann von Vorteil, wenn die Alkylenoxidkomponente der Formel (III) bei der Umsetzungstemperatur gasförmig vorliegt. Dies kann sich insbesondere bei Ethylenoxid oder Propylenoxid vorteilhaft auswirken.

Das erfindungsgemäße Verfahren wird vorzugsweise so durchgeführt, dass das molare Verhältnis der 1- bis 3-basischen Säure zu Alkylenoxid der Formel (II) 1:1 bis 1:2 beträgt. Das molare Verhältnis von Alkylenoxid zu eingesetztem Picolin der Formel (I) beträgt vorzugsweise 1:1 bis 2:1. Selbstverständlich ist die Reaktion auch mit anderen Verhältnissen durchführbar, jedoch führen derartige Verhältnisse zu keinem unerwarteten Vorteil. In einer bevorzugten Verfahrensvariante wird das Picolin der Formel (II) in Wasser oder in einem Gemisch aus Wasser und einem polaren organischen Lösungsmittel als Reaktionsmedium vorgelegt und die Alkylenoxid- und Säurekomponente, vorzugsweise getrennt, zugegeben. In einer bevorzugten Ausführungsform wird so viel Säure unter Bildung des Picoliniumsalzes zugegeben, dass sich ein pH-Wert im Reaktionsmedium von 2 bis 8, vorzugsweise von 4 bis 7 einstellt.

Weiterhin ist es vorteilhaft, die Zugabe von Säure und Alkylenoxid so zu steuern, dass der pH-Wert des Reaktionsgemisches während der Umsetzung in dem genannten pH-Bereich bleibt. Dabei kann die Alkylenoxidzugabe in Abhängigkeit von der Säurezugabe dergestalt erfolgen, dass der pH-Wert im Bereich von 2 bis 8, vorzugsweise von 4 bis 7 gehalten wird.

Besonders bevorzugt erfolgt jedoch die Säurezugabe in Abhängigkeit von der Zugabe des Alkylenoxid (III), wobei die vorteilhaften pH-Bereiche wie oben beschrieben eingehalten werden. Die Ermittlung des pH-Wertes während der Reaktion kann mit pH-Papier oder vorzugsweise mit einer handelsüblichen pH-Elektrode erfolgen. Die Verwendung einer pH-Elektrode hat den Vorteil, dass sie sich in Verbindung mit einer geeigneten Regeleinheit direkt mit einer Zugabe- Einrichtung verwenden lässt, die in Abhängigkeit von dem gewünschten pH-Wert die Zugabe einer der beiden Komponenten wie oben beschrieben steuert. Besonders bevorzugt wird das erfindungsgemäße Verfahren in einer CO₂-Atmosphäre durchgeführt. Dies führt insbesondere bei Verwendung nichtflüchtiger 1- bis 3-basischer Säuren, wie H₂SO₄, zu Verbindungen der Formel (I), die eine besonders hohe Reinheit besitzen.

Die nach dem erfindungsgemäßen Verfahren erhaltenen Salze können beispielsweise durch destillative Entfernung des verwendeten Reaktionsmediums aus dem Reaktionsgemisch isoliert werden.

Besonders bevorzugt ist das erfindungsgemäße Verfahren, wenn keine Halogenhaltigen 1- bis 3-basiche Säuren verwendet werden.

Die Erfindung betrifft weiterhin neue β-Hydroxyalkylpicoliniumsalze der Formel (I), worin R¹ bis R³ die oben genannten Bedeutungen haben und X⁻ für einen Rest eines Äquivalents einer 1- bis 3-basischen Säure außer Halogenid steht. Diese werden im folgenden als Verbindungen der Formel (Ia) bezeichnet. Besonders bevorzugte β-Hydroxyalkylpicoliniumsalze der Formel (Ia) sind solche, worin X⁻ einen Rest eines Äquivalentes von H₂SO₄ bedeutet.

Die erfindungsgemäßen β-Hydroxyalkylpicoliniumsalze besitzen überraschenderweise eine deutlich geringere Korrosivität, als die entsprechenden Chloride, die beispielsweise aus EP-A 176 472 bekannt sind.

Die Erfindung betrifft weiterhin ein Verfahren zur Herstellung kationischer Farbstoffe, das dadurch gekennzeichnet ist, dass man die β-Hydroxyalkylpicoliniumsalze (Ia) oder die nach dem obigen Verfahren erhaltenen β-Hydroxyalkylpicoliniumsalze (I) mit aromatischen oder heterocyclischen Aldehyden umsetzt. Ganz besonders bevorzugt ist dieses Verfahren, ohne dass die nach dem obigen Verfahren erhaltenen β-Hydroxyalkylpicoliniumsalze zwischenisoliert werden.

Als aromatische oder heterocyclische Aldehyde, die für diesen Zweck besonders geeignet sind, sind beispielsweise zu nennen: gegebenenfalls substituierte Benzaldehyde, wie z.B. 4-Dimethylaminobenzaldehyd, 4-Diethylaminobenzaldehyd, 4-Methylbenzylaminobenzaldehyd, 4-Ethylbenzylaminobenzaldehyd,4-Ethylhydroxyethylaminobenzaldehyd, 4-Ethyl-β-chorethylaminobenzaldehyd, 1-Methyl-β-chlorethylaminobenzaldehyd, 4-Dicyanethylaminobenzaldehyd, 4-Ethylcyanethylaminobenzaldehyd, sowie C₁- bis C₄-Alkoxybenzaldehyde, die gegebenenfalls weiter substituiert sein können, wie z.B. 4-Methoxybenzaldehyd, 2,4-Dimethoxybenzaldehyd, 3,4-Dimethoxybenzaldehyd, 4-β-Methoxyethoxybenzaldehyd, sowie heterocyclische Aldehyde, wie z.B. Carbazolaldehyd, Indol-3-aldehyd, 2-Methyl-3-formylindol, 1,2-Dimethyl-3-formylindol, 1 -Methyl-2-phenyl-3-formylindol,1-Benzyl-2-phenyl-3-formylindol, 1,2-Dimethyl-5-methoxy-3-formylindol, 1,2-Dimethyl-5-chlor-3-formylindol, 1,2-Dimethyl-5-methyl-3-formylindol, 1,2-Dimethyl-5,6-dichlor-3-formylindol, 1-Methyl-2-phenyl-5-methoxy-3-formylindol, 1 -Methyl-2-phenyl-5-chlor-3-formylindol, 1-Methyl-2-phenyl-5-methyl-3-formylindol, 1-Methyl-2-phenyl-5,6-dichlor-3-formylindol, 2,2,4-Trimethyl-6-formyltetrahydrochinolin.

Es wurde weiterhin gefunden, dass Farbstoffpräparationen, mit einem Chloridgehalt von kleiner als 0,5 Gew.-%, vorzugsweise kleiner als 0,1 Gew.-%, bezogen auf die Farbstoffpräparation, die mindestens einen kationischen Farbstoff, der aus einem erfindungsgemäßen β-Hydroxyalkylpicoliniumsalz (Ia) hergestellt worden ist, enthalten, deutlich geringere korrosive Eigenschaften besitzen. Dies gilt für feste, also Pulver oder Granulate, besonders aber für flüssige, insbesondere wässrige Präparationen.

Besonders bevorzugt sind die erfindungsgemäßen Farbstoffpräparationen, die im wesentlichen frei von Chloridionen, insbesondere Halogenid-Ionen sind.

Als bevorzugte kationische Farbstoffe dieser Farbstoffpräparationen kommen solche in Frage, die aus nach dem erfindungsgemäßen Verfahren β-Hydroxyalkylpicoliniumsalzen (Ia) hergestellt wurden. Dabei sind solche ganz besonders bevorzugt, die neben diesen kationischen Farbstoffen außerdem den Schwefelsäurehalbester β-Hydroxyalkylhydrogensulfate, insbesondere β-Hydroxyethylhydrogensulfat enthalten.

Im allgemeinen enthalten die erfindungsgemäßen festen Farbstoffpräparationen

| | | |
|---|---|---|
| a) | 50 bis 80 Gew.-% | wenigstens einen aus β-Hydroxyalkylpicoliniumsalz (Ia) hergestellten kationischen Farbstoff, |
| b) | 0 bis 0,5 Gew.-% | Cl⊖ |

Rest Stellmittel, Restfeuchte sowie weitere übliche Zusätze.

Im allgemeinen enthalten die erfindungsgemäßen flüssigen Farbstoffpräparationen

| | | |
|---|---|---|
| a) | 20 bis 50 Gew.-% | wenigstens einen aus β-Hydroxyalkylpicoliniumsalz (Ia) hergestellten kationischen Farbstoff |
| b) | 0 bis 0,5 Gew.-% | Cl⊖ |
| c) | 0 bis 30, insbesondere 5 bis 25 Gew.-% einer organischen Säure, insbesondere Essigsäure | |

sowie gegebenenfalls weiteren für wässrige Präparationen kationsicher Farbstoffe übliche Zusätze.

Als weitere vorteilhafte Zusätze sind die nachfolgend als d) bis f) bezeichneten zu nennen, die in den angegebenen Mengenbereichen vorteilhafte Farbstoffpräparationen liefern:

| | | |
|---|---|---|
| d) | 0 bis 30 Gew.-% | β-Hydroxyalkylhydrogensulfat, insbesondere β-Hydroxyethylhydrogensulfat, |
| e) | 0 bis 15 Gew.-% | Ethylenglykol, |
| f) | 0 bis 15 Gew.-% | Propylenglykol. |

Die Erfindung betrifft weiterhin Farbstoffpräparationen mit einem Chloridgehalt von kleiner 0,5 Gew.-%, insbesondere kleiner 0,1 Gew.-%, bezogen auf die Farbstoffpräparationen, die ein kationischen Farbstoff und β-Hydroxyalkylhydrogensulfat, insbesondere β-Hydroxyethylhydrogensulfat enthalten.

Bevorzugte kationische Farbstoffe, die unter Verwendung des nach dem erfindungsgemäßen Verfahren hergestellten β-Hydroxyalkylpicoliniumsalzen der Formel (I) hergestellt werden, bzw. solche, die bevorzugt in den erfindungsgemäßen Farbstoffpräparationen enthalten sind, entsprechen der Formel (IV) 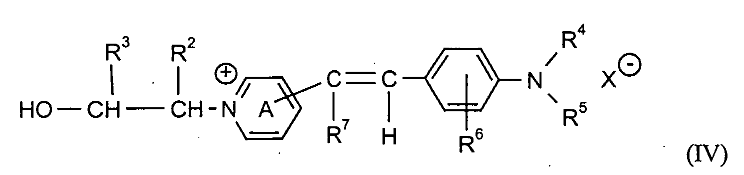 worin
- R², R³, A und X⁻: die oben genannte Bedeutung haben und
- R⁴ und R⁵: unabhängig voneinander C₁-C₄-Alkyl oder Phenyl bedeuten, die gegebenenfalls durch Phenyl, 4-Chlorphenyl, 4-Cyanophenyl oder durch ein oder zwei Substituenten aus der Gruppe OH, C₁-C₄-Alkoxy, Halogen und Cyano substituiert sind,
- R⁶: H, Halogen, CN, Nitro oder C₂-C₄-Alkyl bedeutet und
- R⁷: H oder CN bedeutet.

Bevorzugte kationische Farbstoffe der Formel IV sind solche, die einer der nachfolgenden Formeln entsprechen.

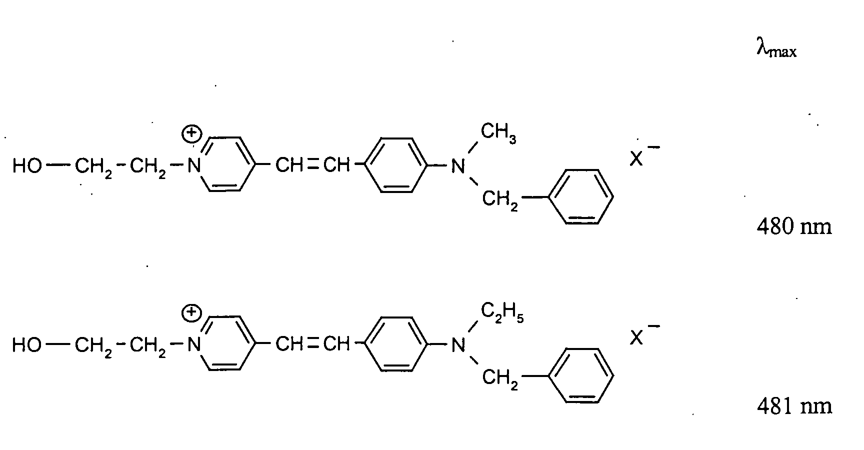

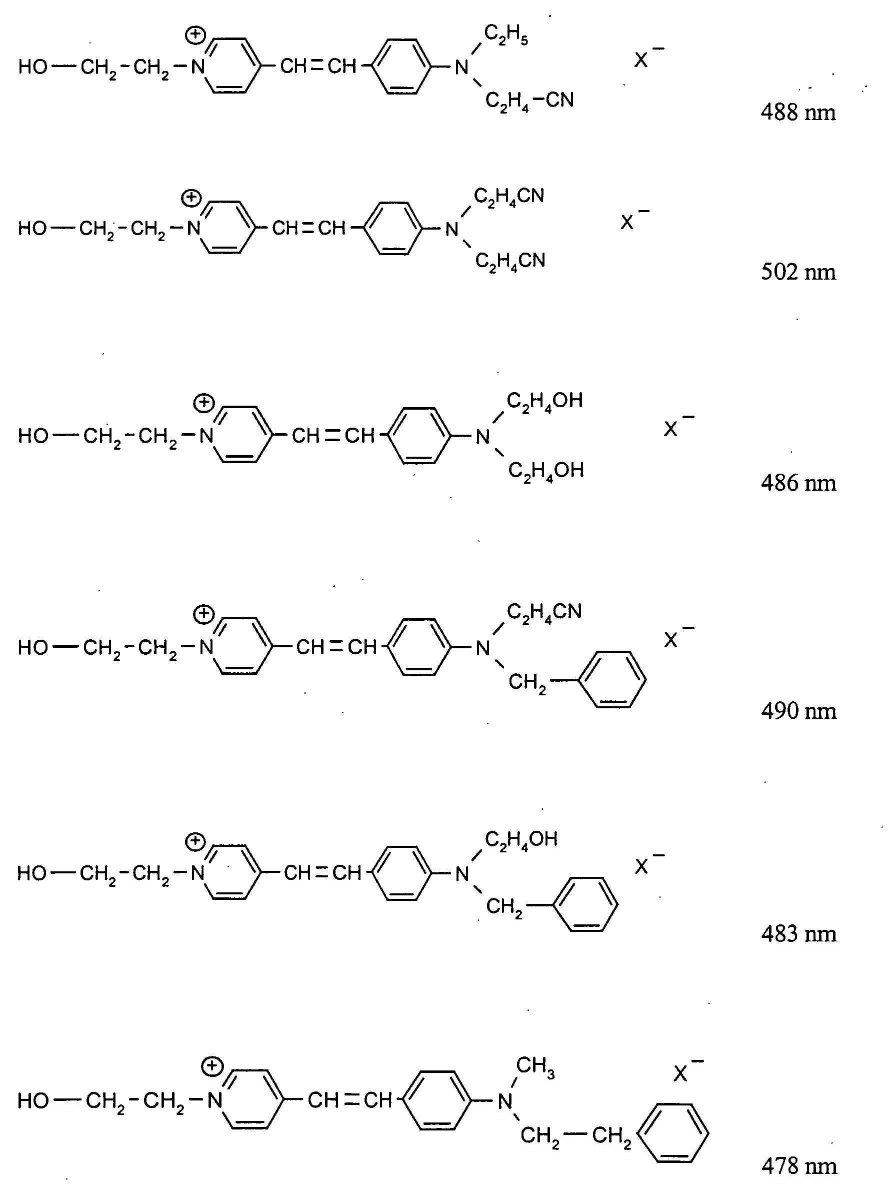

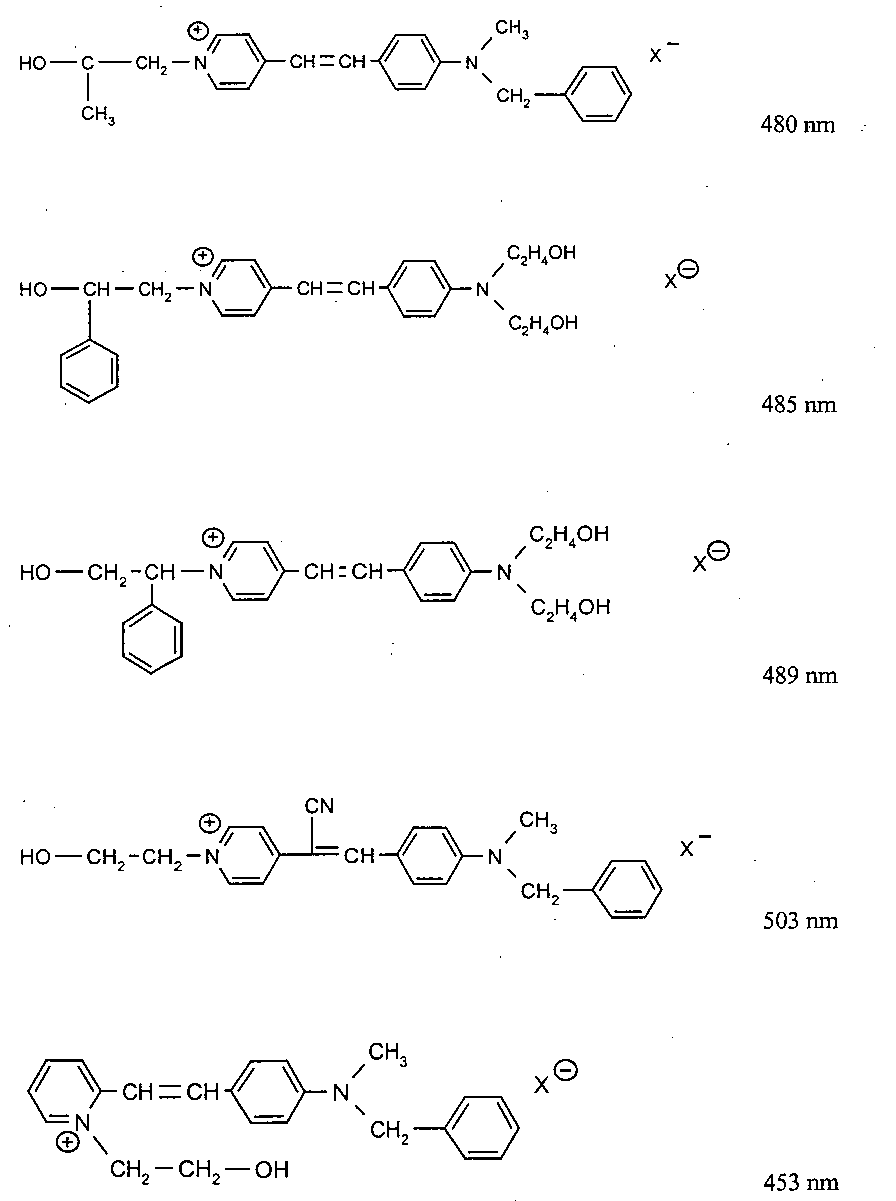

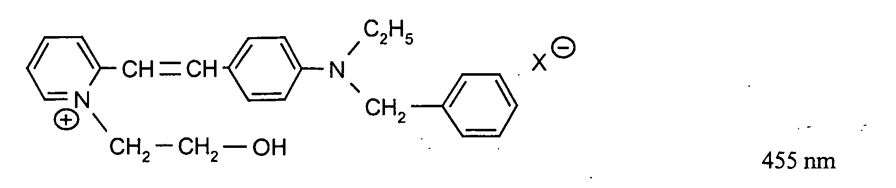

Besonders bevorzugt steht der Aminostyrylrest in ortho- oder para-Stellung, insbesondere in para-Stellung zum Ringstickstoffatom des Picoliniumrests.

### Beispiele

### Beispiel 1

### Apparatur:

Planschliffgefäß mit Bodenablassventil, Destillationsaufsatz, Anschluss zur Dosierung von CO₂, Ethylenoxid und Schwefelsäure, deren Zugabe über einen Titrator geregelt wird.

### Verfahren:

Unter CO₂-Atmosphäre wurden 480 g 4-Methylpyridin, 316,5 g 1,2-Propandiol und 77,4 g Wasser vorgelegt. Mit konzentrierter Schwefelsäure wurde in dieser Mischung ein pH-Wert von 5 eingestellt. Der Ansatz wurde auf 80°C erwärmt. In dieser Apparatur wurde unter gleichzeitiger Dosierung von Schwefelsäure mit Hilfe eines Titrators bei pH 5 so lange Ethylenoxid eingeleitet, bis ein Schwefelsäureverbrauch von 300 g Schwefelsäure erreicht war. Man ließ den pH-Wert auf 6 ansteigen und leitete nun bei diesem pH-Wert weiter Ethylenoxid ein, bis weitere 50 g Schwefelsäure verbraucht waren. Der Ethylenoxidstrom wurde abgestellt. Durch Reaktion des gelösten Ethylenoxids wurden beim Nachrühren noch 7 g Schwefelsäure verbraucht. Der Gesamtschwefelsäureverbrauch lag bei 357 g. Die Dosierung von Schwefelsäure wurde abgestellt, der Ansatz auf Raumtemperatur abgekühlt und abgelassen. Ausbeute: 1518 g einer Reaktionsmischung folgender Zusammensetzung: Picoliniumsalz 45,6 % (bezogen auf ein Molekulargewicht von 138,2), entspricht 97 % der Theorie, 4-Methylpyridin 1,8 %, Nebenprodukt 1,3 %, Rest Wasser sowie 1,2-Propandiol, β-Hydroxyethylhalogensulfat u.a.

### Beispiel 2

Analog Beispiel 1 wurde das Verfahren durchgeführt, anstatt Schwefelsäure jedoch 540 g konzentrierte Essigsäure eingesetzt. Man erhielt 1810 g eines hellgelben Produktgemisches folgender Zusammensetzung:

| | |
|---|---|
| 1,2 % | 4-Methylpyridin |
| 53,5 % | β-Hydroxyethylpicoliniumacetat |
| 1 % | Nebenprodukt |
| Rest: | Wasser, Propandiol, Ethylenglykolsäure, Essigsäureethylenglykolester |

### Beispiel 3

742 Teile γ-Picolin, 119 Teile Wasser und 489 Teile Propylenglykol werden in einem Rührgefäß vorgelegt und dann unter Kühlung mit 199 Teilen 98%iger Schwefelsäure so versetzt, dass die Temperatur auf 60 bis 70°C ansteigt. Das Reaktionsgefäß wird nun zur Entfernung von Luftsauerstoff mit Stickstoff gespült und auf 80°C erwärmt. Nun werden bei 80 bis 90°C 125 Teile Ethylenoxid innerhalb von ca. 1 Stunde zugegeben. Der pH-Wert der Reaktionsmischung soll dabei nicht über 7 ansteigen. Nach der Zugabe von weiteren 200 Teilen Schwefelsäure werden innerhalb von ca. 1 Stunde nochmals 186 Teile Ethylenoxid zugegeben, bis der pH-Wert der Reaktionsmischung 7,5 bis 8 erreicht hat. Es wird noch 1/2 Stunde nachgerührt und dann unter einem Vakuum von 20 mbar bei 90°C solange destilliert, bis kein Destillat mehr übergeht. Es wird ein flüssiges Produkt erhalten, das neben ca. 30 % Propylenoxid ca. 43 % Hydroxyethylpicoliniumsulfat im Gemisch mit 29 % Hydroxyethylpicoliniumhydroxyethylsulfat enthält. (Gehalt an Hydroxyethylpicoliniumkation: 45 bis 50 %, Bestimmung durch HPLC)

### Beispiel 4

Das Verfahren wurde analog Beispiel 1 durchgeführt, jedoch wurde anstatt Schwefelsäure 357 g Phosphorsäure eingesetzt. Man erhält 1545 g eines Produktgemisches der folgenden Zusammensetzung: Picoliniumsalz: 43,6 %, 4-Methylpyridin: 1,7 %, Nebenprodukt: 1,4 % Rest Wasser, Propandiol, Ethylenglykol und Phosphorsäuremono- und diglycolester.

### Beispiel 5

Aus dem Reaktionsgemisch aus Beispiel 1 werden im Wasserstrahlvakuum bis zu einer Badtemperatur von 100°C

124,4 g einer Mischung aus Wasser und 1,2-Propandiol abdestilliert. Man lässt auf 60°C abkühlen und gibt bei dieser Temperatur 957,7 g 4-Ethylbenzylaminobenzaldehyd und 50 ml Piperidin zu. Anschließend wird auf 80°C erwärmt und bei dieser Temperatur 7 Std. nachgerührt. Am nächsten Morgen wird im Wasserstrahlvakuum 4 Stunden abdestilliert. Man erhält 2467,7 g nicht eingestellten Farbstoffs. Durch Zugabe von 730 g Eisessig und 1459 g Wasser wird die Farbstärke eingestellt.

Ausbeute: 4656 g Flüssigeinstellung

### Beispiel 6

Ein analog zu Beispiel 5 hergestellter kationischer Farbstoff zu dessen Herstellung jedoch Hydroxyethylpicoliniumchlorid verwendet wurde, wurde in Form seiner wässrigen Farbstoffpräparation in einem Korrosionstest eingesetzt, bei dem geprüft werden sollte, ob V2A- bzw. V4A-Stahl-Werkstoffe in Gegenwart dieses Farbstoffes korrodieren.

Die Ergebnisse der Stromdichte Potential-Messungen ergaben, dass die getesteten Werkstoffe korrodierten.

### Beispiel 7

Analog Beispiel 6 wurden die Werkstoffe in Gegenwart einer wässrigen Farbstoffpräparationen des chlorid-freien Farbstoffes aus Beispiel 5 auf Korrosion getestet. Die Ergebnisse der Stromdichte-Potential-Messungen ergaben, dass die getesteten V2A- bzw. V4A-Stahl Werkstoffe nicht korrodierten.

## Patentansprüche

1. β-Hydroxyalkylpicoliniumsalze der Formel (Ia) 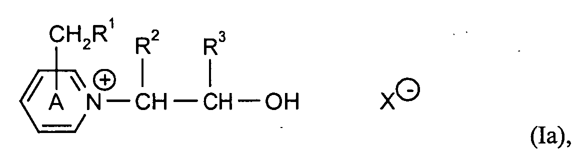 worin
der Ring A gegebenenfalls weitere Substituenten trägt und/oder mit carbo- oder heterocyclischen Ringen kondensiert ist,
R¹ für H, Cyano, C₁-C₄-Alkoxycarbonyl oder gegebenenfalls substituiertes C₁-C₄-Alkyl steht,
R² und R³ unabhängig voneinander für H, gegebenenfalls substituiertes C₁-C₄-Alkyl oder gegebenenfalls substituiertes Phenyl stehen und
X⁻ für den Rest eines Äquivalents einer 1- bis 3-basischen Säure, ausgenommen Halogenid, steht.

2. Verfahren zur Herstellung von kationischen Farbstoffen, **dadurch gekennzeichnet, dass** β-Hydroxyalkylpicoliniumsalze gemäß Anspruch 1 mit aromatischen oder heteroaromatischen Aldehyden umgesetzt werden.

3. Farbstoffpräparationen mit einem Chloridgehalt von weniger als 0,5 Gew.-%, bezogen auf die Farbstoffpräparation, die wenigstens einen kationischen Farbstoff, der aus β-Hydroxyalkylpicoliniumsalzen gemäß Anspruch 1 hergestellt wurde, enthalten.

4. Farbstoffpräparationen gemäß Anspruch 3, **dadurch gekennzeichnet, dass** sie außerdem β-Hydroxyethylhydrogensulfat enthalten.

5. Farbstoffpräparationen mit einem Chloridgehalt von kleiner 0,5 Gew.-%, insbesondere kleiner 0,1 Gew.-%, bezogen auf die Farbstoffpräparationen, die ein kationischen Farbstoff und β-Hydroxyalkylhydrogensulfat, insbesondere β-Hydroxyethylhydrogensulfat enthalten.
